# EUROPEAN PATENT SPECIFICATION

(11) **EP 1 828 218 B1**
(45) Date of publication and mention of the grant of the patent: **30.04.2014**
(21) Application number: 05850457.2
(22) Date of filing: 15.12.2005
(51) Int. Cl.: C07H 21/00, C07D 233/54

(54) **SYNTHESIS OF PHOSPHITYLATED COMPOUNDS USING A QUATERNARY HETEROCYCLIC ACTIVATOR**
HERSTELLUNG PHOSPHITYLIERTER VERBINDUNGEN UNTER VERWENDUNG EINES QUATERNÄREN HETEROZYKLISHEN AKTIVATORS
SYNTHÈSE DES COMPOSÉS PHOSPHITYLÉS

(30) Priority: 15.12.2004 US 636152 P; 15.12.2004 EP 04106599
(43) Date of publication of application: 05.09.2007
(73) Proprietor: Girindus AG, 51427 Bergisch Gladbach (DE)
(72) Inventor: LANGE, Meinolf, 72181 Starzach-Felldorf (DE); SCHÖNBERGER, Andreas, 38539 Müden/Aller (DE); KIRCHHOFF, Christina, 33803 Steinhagen (DE); GRÖSSEL, Olaf, 33790 Halle/Westfalen (DE); OMELCENKO, Nadja, 33790 Halle/Westfalen (DE); HOHLFELD, Andreas, 33790 Halle/Westfalen (DE); LINK, Fritz, 51427 Bensberg (DE)
(74) Representative: Mross, Stefan P.M.
(86) International application number: PCT/EP2005/056815
(87) International publication number: WO 2006/064039

(56) References cited:
- EP-A- 0 906 917
- WO-A-99/62922
- HAYAKAWA Y ET AL: "Acid/azole complexes as highly effective promoters in the synthesis of DNA and RNA oligomers via the phosphoramidite method" JOURNAL OF THE AMERICAN CHEMICAL SOCIETY, vol. 123, no. 34, 29 August 2001 (2001-08-29), pages 8165-8176, XP001188947 ISSN: 0002-7863 cited in the application
- ARNOLD ET AL: "Automated Chloridite and Amidite Synthesis of Oligodeoxyribonucleotides on a Long Chain Support using Amidine Protected Purine Nucleosides" COLLECTION OF CZECHOSLOVAK CHEMICAL COMMUNICATIONS, vol. 54, 1989, pages 523-532, XP001161127 ISSN: 0010-0765 cited in the application
- IWAI S ET AL: "Benzimidazolium triflate-activated synthesis of (6-4) photoproduct-containing oligonucleotides and its application" NUCLEIC ACIDS RESEARCH, vol. 27, no. 11, 1 June 1999 (1999-06-01), pages 2299-2303, XP001207111 ISSN: 0305-1048
- HAYAKAWA ET AL: "Benzimidazolium triflate as an efficient promoter for nucleoside synthesis via the phosphoramidite method" JOURNAL OF ORGANIC CHEMISTRY, vol. 61, 1996, pages 7996-7997, XP002110027 ISSN: 0022-3263 cited in the application
- HAYAKAWA Y ET AL: "Facile synthesis of oligodeoxyribonucleotides via the phosphoramidite method without nucleoside base protection" JOURNAL OF THE AMERICAN CHEMICAL SOCIETY, vol. 120, no. 48, 1998, pages 12395-12401, XP001188946 ISSN: 0002-7863 cited in the application
- BEAUCAGE S L ET AL: "Tetrahedron Report number 309: Advances in the synthesis of oligonucleotides by the phosphoramidite approach" TETRAHEDRON, vol. 48, no. 12, 1992, pages 2223-2311, XP000915225 ISSN: 0040-4020

## Description

### Field of the invention

The present invention relates to methods for preparing phosphitylated compounds using specific activators, especially to the synthesis of phosphoramidites.

### Background of the invention

Oligonucleotides are key compounds in life science having important roles in various fields. They are for example used as probes in the field of gene expression analysis, as primers in PCR or for DNA sequencing.

Furthermore, there are also a number of potential therapeutic applications including i.e. antisense oligonucleotides.

A number of chemical modifications have been introduced into oligonucleotides to increase their usefulness in diagnostics, as research agents and as therapeutic agents, for example to stabilize against nucleases.

Synthesis of oligonucleotides can be accomplished using both solution phase and solid phase methods. The currently preferred method is via solid-phase synthesis wherein an oligonucleotide is prepared on a solid support and the oligonucleotide grows by sequential addition of nucleotides.

The growing number of applications requires larger quantities of oligonucleotides; therefore, there is an ongoing need for developing improved synthetic method.

For a general overview, see for example "Antisense - From Technology to Therapy" Blackwell Science (Oxford, 1997).

One prominent type of building blocks in the synthesis of oligonucleotides are phosphoramidites; see for example S.L. Beaucage, M. H. Caruthers, Tetrahedron Letters 1859 (1981) 22. These phosphoramidites of nucleosides, deoxyribonucleosides and derivatives of both are commercially available. In normal solid phase synthesis 3'-O-phosphoramidites are used but in other synthetic procedures 5'-O and 2'-O-phosphoramidites are used, too. One step in the preparation of these nucleosides phosphoramidites is the phosphitylating of the (protected) nucleosides. Most commonly, the hydroxyl group and amino groups and other functional groups present in the nucleoside are protected prior to phosphitylating the remaining 3'-, 5'- or 2'-O hydroxyl group. Several routes are known for the preparation of monomeric (nucleosides) and polymeric (nucleotides or oligonucleotides) phosphoramidites. The known methods result very often in problems of chemistry or safety. For the usage of this chemistry for larger batches synthesis (100 kg-1000 kg) the cost effectiveness has to be improved.

Traditionally, phosphitylation of nucleosides is performed by treatment of the protected nucleosides with a phosphitylating reagent such as chloro-(2-cyanoethoxy)-N,N-diisopropylaminophosphine which is very reactive and does not require an activator or 2-cyanoethyl-N,N,N',N'-tetraisopropylphosphorodiamidite (bis-phos or bis-amidite reagent) which requires an activator.

The activator most commonly used in phosphitylation reaction is 1*H*-tetrazol.

There are inherent problems with the use of 1*H*-tetrazol, especially when performing larger scale synthesis. For example, 1*H*-tetrazol is known to be explosive and toxic. According to the material safety data sheet (MSDS) 1*H*-tetrazol (1*H-*tetrazol, 98%) can be harmful if inhaled, ingested or absorbed through the skin.

Furthermore, 1*H*-tetrazole is expensive. Especially in large scale synthesis it has a considerable impact on the synthesis costs of the oligonucleotides.

The MSDS also states that 1*H*-tetrazol can explode if heated above its melting temperature of 155°C and may form very sensitive explosive metallic compounds. In the case of the large scale synthesis in vessel 1*H*-tetrazol would include a major danger of human and surrounding.

In addition, it is known that 1*H*-tetrazol requires special handling during its storage, use and disposal.

1*H*-tetrazol and the related derivatives, e.g. 5-ethylthio-1*H*-tetrazole, 5-benzylthio-1*H*-tetrazole have also the potential for the decomposition of the target molecule. Therefore the cleavage of acid sensitive protective group were reported in different publications (Krotz et al, Tetrahedron Letters, 1997, 38, 3875).

Inadvertent deprotection of the acid labile protective group are also known for the use of chloro-(2-cyanoethoxy)-N,N-diisopropylaminophosphine. Beside the tendency of cleaving the used protective groups this phosphitylating agent will result in larger amounts of the 3'-3' isomers. The resulting amidites have to be purified by a time and cost intensive chromatography step.

Especially in the application for the phosphitylation of oligomeric phosphoramidites the known methods resulted mostly in decomposition or complex mixtures of the target molecule and by products.

The usage of bis-phos with certain activators is generally known for monomeric nucleoside amidites, but in the case of oligonucleotides the low reactivity made this approach very complicated.

The low reactivity resulted also in long reaction time (2-6 h). Avoiding the long reaction time will require the usage of a massive excess of phosphitylation agent and activator. At the end this kind of reaction management will also require additional purification steps.

EP 0 906 917 A2 and Hayakawa et al., J. Am. Chem. Soc. 120 (1998) 12395-12401 disclose the use of imidazolium triflate for the synthesis of phosphoramidites. Yield and purity of the described synthesis could not be repeated.

In addition the process of Hayakawa will apply with the usage of an activator, which was prepared, isolated and purified separately. After the purification of the water sensitive activator it is necessary to store this activator under totally dry conditions.

The sensitivity and the low reactivity of this activator will result in a complicate handling, which is difficult for the large scale synthesis of amidites.

In all experiments with this activator of Hayakawa, the resulting amidites have to be purified by a cost intensive chromatographic step.

However, in all cases the result of the phosphitylation reaction was incomplete and inefficient, and therefore a purification step is always a major requirement.

The phosphitylation of sensitive oligonucleotides ended mostly in decomposition.

The yields and purity of the described synthesis could not be repeated, because the used imidazolium triflates have a high nucleophilic character and a high hydroscopic tendency. These proprieties will end up with major quantities of decomposition and hydrolysis. The described activators were isolated and used in their pure form.

This method for the synthesis of amidites requires a flash chromatography for the purification of the target compound.

In addition Hayakawa used the compound for the formation of the internucleotide bond (condensation of the amidite with a nucleoside).

Hayakawa et al., J. Org. Chem. 61 (1996) 7996-7997 dislcose the use of benzimidazolium triflate for condensation of a phosphoramidite with a nucleoside.

Hayakawa et al., J. Am. Chem. Soc. 123 (2001) 8165-8176 disclose the use of acid/azole complexes for condensation of a phosphoramidite with a nucleoside.

Arnold et al., Collect. Czech. Chem. Commun. 54 (1989) 523-532 disclose automated chloridite and amidite synthesis of oligodeoxyribonucleotides, and inter alia the use of 1-methylimidazole in condensation of a phosphoramidite with a nucleoside.

### Summary of the invention

It is an object of the present invention to provide a method for preparing phosphitylated compounds overcoming at least some of the drawbacks of prior art.

It is a further object of the invention to provide an activator having improved properties when compared to activators of prior art.

It is a further object of the invention to provide an activator/additive mixture having improved properties when compared to activators of prior art. In one aspect, the present invention provides a method for preparing a phosphitylated compound comprising the step of:
- reacting a hydroxyl containing compound with a phosphitylating agent in the presence of an activator having the formula I 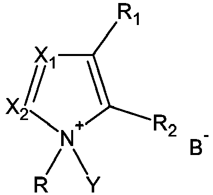 wherein
   R = alkyl, cycloalkyl, aryl, aralkyl, heteroalkyl, heteroaryl
   R₁, R₂ = either H or form a 5 to 6-membered ring together
   X₁, X₂ = independently either N or CH
   Y = H or Si(R₄)₃, with R₄= alkyl, cycloalkyl, aryl, aralkyl, heteroalkyl, heteroaryl
   B = deprotonated acid.

The activator can be used stoichiometrically or catalytically (3 to 50 mole%, preferably 10 to 30 mole%) or in excess (up to 300 mole%).

In a preferred embodiment, the activator has a formula selected from the group consisting of 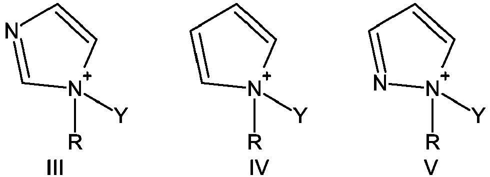 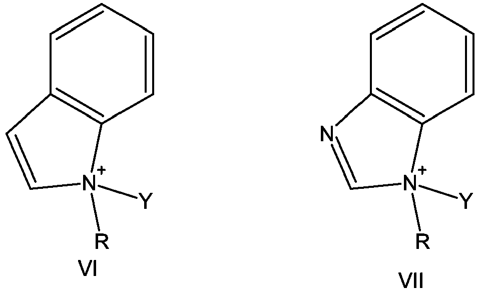 wherein
Y is defined as above
R is methyl, phenyl or benzyl.

The preparation of these activators is for example described in Hayakawa et al, J. Am. Chem. Soc. 123 (2001) 8165-8176.

In one embodiment the activator is used in combination with an additive. Additives can be selected from the unprotonated form of the compounds having formula I and other heterocyclic bases for example pyridine. Suitable ratios between the activator and the additive are 1:1 to 1:10.

In one preferred embodiment, the activator can be prepared following an "in situ" procedure. In this case the activator will not be isolated, which resulted in improved results of the reaction. Hydrolysis or decomposition of the target molecule is suppressed.

For a high yielding phosphitylation in 3'- and/or 5'-position of oligonucleotides (di, tri, tetra, penta, hexa, hepta and octamers), the in-situ preparation of the activator and the combination with an additive is preferred.

As described above phosphitylation is especially useful in the synthesis of oligonucleotides and the building block phosphoramidites. Therefore, in a preferred embodiment, the hydroxyl containing compound comprises a sugar moiety for example a nucleoside or an oligomer derived there from. Such nucleosides are for example adenosine, cytosine, guanosine and uracil, desoxyadenosine, desoxyguanosine, desoxythymidin, desoxycytosine and derivatives thereof, optionally comprising protective groups.

The method of the present invention is especially useful for phosphitylating oligonucleotides (di, tri, tetra, penta, hexa, hepta and octamers). Such phosphitylated oligonucleotides are used for example for the synthesis of large oligonucleotides through a fragment condensation concept.

Normally, they will be suitably protected on their heterocyclic functionality and on their hydroxyl bearing groups except of the one that should be phosphitylated. Typically, dimethoxytrityl, monomethoxytrityl or silyl containing protective groups (e.g. TBDMS) are used as protective groups for the 5'-OH-group, allowing phosphitylation of the 3'-OH group.

Also the 3'-OH group can be protected with a protective group (LEV, TBDMS etc.) and the deprotected 5'-OH will allow the 5'-O-phosphitylation of nucleosides or nucleotides.

The methods of phosphitylation can be used for the synthesis of 3'- or 5'- phosphoramidites with identical results.

The resulting target molecule of the phosphitylation reaction is in one embodiment a phosphoramidite and has the structure: 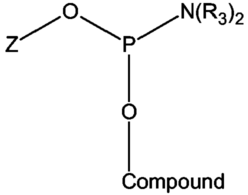 Z represents a leaving group e.g. CH₃, C₂H₅, CH₂C₆H₅, -CH₂CH₂CN, - CH₂CH=CHCH₂CN, para-CH₂C₆H₄CH₂CN, -(CH₂)₂₋₅N(H)COCF₃, - CH₂CH₂Si(C₆H₅)₂CH₃, or -CH₂CH₂N(CH₃)COCF₃ and wherein R₃ is alkyl having from 1 to about 6 carbons; or R₃ is a heterocycloalkyl or heterocycloalkenyl ring containing from 4 to 7 atoms, and having up to 3 heteroatoms selected from nitrogen, sulphur, and oxygen, and "compound" is the rest of hydroxy containing compound, e.g. a nucleoside, nucleotide or an oligonucleotide.

In this case the P(III) atom is connected to two oxygen atoms (or forming two P-O bonds) and one nitrogen atom (forming one P-N bond), which belongs to an amino group, preferentially diisopropyl amine, diethylamine or other secondary amines.

The condensation reaction of the phosphoramidite with an other hydroxyl group of an other molecule (compound A) will result in a phosphite triester with the structure:

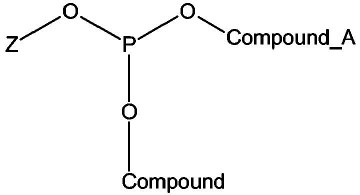

In this case the P(III) atom has connections to three oxygen atoms (forming three P-O bonds) and no bond to nitrogen.

In general, the phosphitylating agent can be the same as in phosphitylating reactions using 1H-tetrazole.

In a preferred embodiment, it has the formula 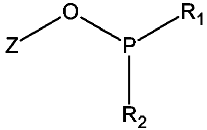 wherein Z represents a leaving group e.g. CH₃, C₂H₅, CH₂C₆H₅, -CH₂CH₂CN, - CH₂CH=CHCH₂CN, para-CH₂C₆H₄CH₂CN, -(CH₂)₂₋₅N(H)COCF₃, - CH₂CH₂Si(C₆H₅)₂CH₃, or -CH₂CH₂N(CH₃)COCF₃ and R₁ and R₂ are independently secondary amino groups N(R₃)₂, wherein R₃ is alkyl having from 1 to about 6 carbons; or R₃ is a heterocycloalkyl or heterocycloalkenyl ring containing from 4 to 7 atoms, and having up to 3 heteroatoms selected from nitrogen, sulphur, and oxygen.

A typical phosphytilating agent is 2-cyanoethyl-N,N,N',N'-tetraisopropylphosphorodiamidite.

Other preferred phosphitylating reagents are oxazaphospholidine derivatives as described in N. Ok et al., J. Am. Chem. Soc. 2003, 125, 8307 to 8317 incorporated by reference. This phosphytilating agent allows the synthesis of oligonucleotides wherein the internucleotide bond can be converted to phosphothioates in a stereo selective manner. Such diastereoselective synthesized internucleotidic phosphothioate linkages have promising impact on the use of phosphothioates as antisense drugs.

Suitable examples of depronated acids B⁻ are triflate, trifluoroacetate, dichloroacetate, mesyl, tosyl, o-chlorophenolate. Acids with a pKa below 4.5 are preferred. Preferably, they have a low nucleophilicity.

In one embodiment, the reaction is conducted in the presence of molecular sieves or other water binding reagents. In general water should be excluded or fixed by a selected drying media during the reaction.

It is either possible to combine the activator of the present invention with the phosphitylating agent and add the hydroxyl component later. It is also possible to combine the activator with the hydroxyl containing compound and add the phosphitylating agent thereafter.

In the case of using an additive, the activator is mixed with the hydroxy component before the phosphitylating agent is added.

For the "in situ" generation of the activator the selected acid is preferably added after the addition of the additive under controlled reaction temperature.

The phosphitylating agent can be added before the addition of the selected acid or thereafter.

In relation to the addition of acid and phosphitylating agent the nucleoside component can be added at the end or at the beginning.

In a preferred embodiment, the corresponding base of the activator, the hydroxyl containing compound, and the phosphitylating agent are combined and the acid is added to start the reaction.

A further object of the invention is the use of an activator having formula I 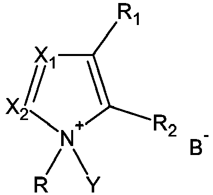 wherein
R = alkyl, cycloalkyl, aryl, aralkyl, heteroalkyl, heteroaryl
R₁, R₂ = either H or form a 5 to 6-membered ring together
X₁, X₂ = independently either N or CH
Y = H or Si(R₄)₃, with R₄= alkyl, cycloalkyl, aryl, aralkyl, heteroalkyl, heteroaryl B = deprotonated acid
as an activator for phosphitylating hydroxyl containing compounds with a phosphitylating agent.

A further object of the invention is the combination of the activator and a non-protonated base (additive), which will form a equilibrium between both species. The resulting equilibrium shows improved properties when compared with activators of prior art.

Especially, in conjunction with the use of acetone the activator/catalyst will not show the known side reactions (decomposition or formation of the 3'-3' or 5'-5'homologue). Acetone has also the ability to dissolve educts and reagents.

According to prior art, in the case of longer reaction times the liberation of diisopropylamine and the presence of activated Bis-Phos results in decomposition of the target compound (detritylation, CE-cleavage, depurination or cleavage of other protective groups.) The presence of acetone and the specific formulation of the activator reduces these tendencies.

The presence of acetone quenches the activity of any amount of diisopropylamine (DIPA), which is liberated during the phosphitylation process. In accordance with the process according to the invention. This can be used for the phosphitylation of shorter and longer oligonucleotides with similar results (no decomposition). Other ketone compounds having the formula Rₓ-C(=O)-R_{y} wherein Rₓ and R_{y} are independently C₁-C₆ alkyl or form an cycloalkyl together can also be used as long as they are able to form enolates in the presence of, e.g. amines has a CH₂-group in the α-position.

In addition the usage of acetone allows longer reaction time without the cleavage of the 5'-O-protective group. In both cases the usage of acetone will protect the different protective groups, and avoid the known tendency of depurination.

Acetone has also a better profile of toxicity and improved environmental properties compared to, e.g. acetonitrile, and is inexpensive.

The combination of the activator with a certain amount of additives supports a higher efficiency of the phosphitylation process of longer and sensitive oligonucleotides (3' or 5' deprotected).

Typically the reactivity of the reagent increases to finalize the synthesis after 2-5 min.

By using the methods of the present invention an additional purification step will not be necessary.

The resulting monomer and oligomer amidites can be used for solid and solution phase synthesis of oligonucleotides.

The activator or activator/additive combination is especially useful in the synthesis of adenosine phosphoramidite, cytosine phosphoramidite, guanosine phosphoramidite and uracil phosphoramidite, desoxyadenosine phosphoramidite, desoxyguanosine phosphoramidite, desoxythymidin phosphoramidite, desoxycytosine phosphoramidite as well as oligonucleotide phosphoramidates having the formula Xₙ, wherein each X is selected from A, dA, C, dC, G, dG, U, dT and n = 2 to 30, preferably 2 to 12, more preferably 2 to 8 or 2 to 6 and derivatives thereof comprising protective groups.

As used herein oligonucleotides cover also oligonucleosides, oligonucleotide analogs, modified oligonucleotides, nucleotide mimetics and the like in the form of RNA and DNA. In general, these compounds comprise a backbone of linked monomeric subunits where each linked monomeric subunit is directly or indirectly attached to a heterocyclic base moiety. The linkages joining the monomeric subunits, the monomeric subunits and the heterocyclic base moieties can be variable in structure giving rise to a plurality of motives for the resulting compounds.

Modifications known in the art are the modification of the heterocyclic bases, the sugar or the linkages joining the monomeric subunits. Variations of internucleotide linkages are for example described in WO 2004/011474, starting at the bottom of page 11, incorporated by reference.

Typical derivatives are phosphorthioates, phosphorodithioates, methyl and alkyl phosphonates and phosphonoaceto derivatives.

Further typical modifications are at the sugar moiety. Either the ribose is substituted by a different sugar or one or more of the positions are substituted with other groups such as F, O-alkyl, S-alkyl, N-alkyl. Preferred embodiments are 2'-methyl and 2'-methoxyethoxy. All these modifications are known in the art.

Concerning the heterocyclic base moiety, there are a number of other synthetic bases which are used in the art, for example 5-methyl-cytosine, 5-hydroxy-methyl-cytosine, xanthin, hypoxanthin, 2-aminoadenine, 6- or 2-alkyl derivatives of adenine and guanine, 2-thiouracyl. Such modifications are also disclosed in WO 2004/011474 starting from page 21.

When used in synthesis these bases normally have protecting groups, for example N-6-benzyladenine, N-4-benzylcytosine or N-2-isobutyryl guanine. In general, all reactive groups which are not intended to react in a further reaction have to be protected, especially the hydroxyl groups of the sugar.

In embodiments related to the synthesis of oligonucleotide phosphoramidite it is useful to conduct the reaction in the presence of acetone or other ketones such as acetone, butanone, pentanone, hexanone, cyclohexanone that can be either used as a reaction media or as a co-solvent for other solvents.

The invention is further explained by the following non-limiting examples.

### Example 1

### Synthesis of 5'-O-DMTr-T-3'-O-phosphoramidite using Methyl-imidazolium-trifluoroacetate

5.0 g 5'-O-DMTr-T-3'-OH (9.2 mmol, 1.0 eq.) and 2.34 g Methyl-imidazolium-trifluoroacetate (11.9 mmol, 1.3 eq.) are dissolved in 100 ml dichloromethane and 3 g molecular sieve 3A is added and the mixture stirred for 10 min. 3.8 ml 2-Cyanoethyl N,N,N',N'-tetraisopropylphosphordiamidite (11.9 mmol, 1.3 eq.) is added. The reaction is complete after 2 h. Yield (determined by HPLC): 95%.

### Example 2

### Synthesis of 5'-O-DMTr-dG^{iBu}-3'-O-phosphoramidite using Benzyl-imidazolium-trifluoroacetate

322 mg Methyl-imidazolium-trifluoroacetate (1.64 mmol, 1.05 eq.) and 1.0 g 5'-O-DMTr-dG^{iBu}-3'-OH (1.56 mmol, 1.0 eq.) are dissolved in 10 ml dichloromethane and 500 mg molecular sieve 3Å is added. 30 min later 0.52 ml 2-Cyanoethyl N,N,N',N'-tetraisopropylphosphordiamidite (1.64 mmol, 1.05 eq.) and 0.1 ml acetone is added to the stirred solution. The reaction is complete after 30 min. Yield (determined by HPLC): 74%.

### Example 3

### Synthesis of 5'-O-DMTr-dC^{Bz}-3'-O-phosphoramidite using Methyl-imidazolium-trifluoroacetate

9.51 g 5'-O-DMTr-dC^{Bz}-3'-OH (15 mmol, 1.0 eq.) are dissolved in 80 ml acetone and 80 ml acetonitrile. 6.17 g Methyl-imidazolium-trifluoroacetate (32 mmol, 2.1 eq.) and 9.64 g 2-Cyanoethyl N,N,N',N'-tetraisopropylphosphordiamidite (32 mmol, 2.1 eq.) is added to the stirred solution. The reaction is complete after 30 min. 500 ml ethylacetate are added, the solution is extracted twice with 250 ml NaHCO₃-solution and with 250 ml brine. The organic layer is dried with MgSO₄ and evaporated to dryness. The residue is dissolved in 40 ml dichloromethane, 250 ml pentane are added, the supernatant is decanted and the residue is dried under reduced pressure to form a colorless foam. Yield (12.0 g, 14.4 mmol): 96%, purity (determined by HPLC): 93%.

### Example 4

### Synthesis of 5'-O-DMTr-dA^{Bz}-3'-O-phosphoramidite using Benzyl-imidazolium-trifluoroacetate

38 mg Benzyl-imidazolium-trifluoroacetate (0.14 mmol, 1.5 eq.) is dissolved in 5 ml acetonitrile and 300 mg molecular sieve 3Å is added. 145 µl 2-Cyanoethyl N,N,N',N'-tetraisopropylphosphordiamidite (0.46 mmol, 5.0 eq.) is added. 30 min later 61 mg 5'-O-DMTr-dA^{Bz}-3'-OH (0.09 mmol, 1.0 eq.) is added and the solution is stirred over night. The reaction is complete after 17 h. Yield (determined by HPLC): 91%.

### Example 5

### Synthesis of 5'-O-DMTr-dC^{Bz}-3'-O-phosphoramidite using a catalytic amount of Methyl-imidazolium-trifluoroacetate

500 mg 5'-O-DMTr-dC^{Bz}-3'-OH (0.79 mmol, 1.0 eq.) are dissolved in 18 ml dichloromethane and 1 ml DMF, 3 g molecular sieve 3Å is added. 50 mg Methyl-imidazolium-trifluoroacetate (0.17 mmol, 0.2 eq.) and 276 µl 2-Cyanoethyl N,N,N',N'-tetraisopropylphosphordiamidite (0.87 mmol, 1.1 eq.) is added to the stirred solution. The reaction is complete after 24 h. Yield (determined by HPLC): 89%.

### Example 6

### Synthesis of 5'-O-DMTr-dG^{iBu}-3'-O-phosphoramidite using a catalytic amount of Benzyl-imidazolium-trifluoroacetate

5 mg Benzyl-imidazolium-trifluoroacetate (0.02 mmol, 0.2 eq.) is dissolved in 5 ml acetonitrile and 300 mg molecular sieve 3Å is added. 145 µl 2-Cyanoethyl N,N,N',N'-tetraisopropylphosphordiamidite (0.46 mmol, 5.0 eq.) is added to the stirred solution. 1 h later 60 mg 5'-O-DMTr-dG^{iBu}-3'-OH (0.09 mmol, 1.0 eq.) is added and the solution is stirred over night. The reaction is complete after 48 h. Yield (determined by HPLC): 90%.

### Example 7

### Synthesis of 5'-O-DMTr-T-3'-O-phosphoramidite using a catalytic amount of Benzyl-imidazolium-trifluoroacetate

50 mg Benzyl-imidazolium-trifluoroacetate (0.18 mmol, 0.18 eq.) and 500 mg 5'-O-DMTr-T-3'-OH (0.92 mmol, 1.0 eq.) are dissolved in 28 ml dichloromethane and 3 g molecular sieve 3Å is added. 350 µl 2-Cyanoethyl N,N,N',N'-tetraisopropylphosphordiamidite (1.0 mmol, 1.1 eq.) is added to the stirred solution. The reaction is complete after 25 h. Yield (determined by HPLC): 90%.

### Example 8

### Synthesis of 5'-O-DMTr- T-P(S)-dC^{Bz}-3'-O-phosphoramidite using Methylimidazolium-trifluoroacetate

100 mg 5'-O-DMTr-T-P(S)-dC^{Bz}-3'-OH (0.10 mmol, 1.0 eq.) and 24.4 mg Methylimidazolium-trifluoroacetate (0.11 mmol, 1.1 eq.) are dissolved in 10 ml dichloromethane, 200 mg molecular sieve 4Å is added. 32 µl 2-Cyanoethyl N,N,N',N'-tetraisopropylphosphordiamidite (0.10 mmol, 1.0 eq.) is added to the stirred solution. The reaction is complete after 24 h. Yield (determined by HPLC): 60%.

### Example 9

### Synthesis of 5'-O-DMTr-dC^{Bz}-P(S)-dG^{iBu}-3'-O-phosphoramidite using Methylimidazolium-trifluoroacetate

100 mg 5'-O-DMTr-dC^{Bz}-P(S)-dG^{iBu}-3'-OH (0.09 mmol, 1.0 eq.) and 17.8 mg Methyl-imidazolium-trifluoroacetate (0.09 mmol, 1.0 eq.) are dissolved in 10 ml dichloromethane, 200 mg molecular sieve 4Å is added. 28 µl 2-Cyanoethyl N,N,N',N'-tetraisopropylphosphordiamidite (0.09 mmol, 1.0 eq.) is added to the stirred solution. The reaction is complete after 3 h. Yield (determined by HPLC): 56%.

### Example 10

### Synthesis of 5'-O-DMTr-dG^{iBu}-P(O)-dG^{iBu}-3'-O-phosphoramidite using Methylimidazolium-trifluoroacetate

106 mg 5'-O-DMTr-dG*^{iBu}*-P(O)-dG*^{iBu}*-3'-OH (0.10 mmol, 1.0 eq.) and 30 mg Methyl-imidazolium-trifluoroacetate (0.15 mmol, 1.5 eq.) are dissolved in 10 ml acetone, 500 mg molecular sieve 3Å is added. After 30 min 34 µl 2-Cyanoethyl N,N,N',N'-tetraisopropylphosphordiamidite (0.11 mmol, 1.1 eq.) is added to the stirred solution. The reaction is complete after 4 h. Yield (determined by HPLC): 55%.

### Example 11

### Synthesis of 5'-O-DMTr-T-P(S)-dC^{Bz}-P(S)-T-P(S)-dC^{Bz}-P(S)-dez-P(S)-dC^{Bz}-3'-O-phosphoramidite using Methyl-imidazolium-trifluoroacetate

10 mg 5'-O-DMTr-T-P(S)-dC^{Bz}-P(S)-T-P(S)-dC^{Bz}-P(S)-dC^{Bz}-P(S)-dC^{Bz}-3'-OH (3.6 µmol, 1.0 eq.) and 1.4 mg Methyl-imidazolium-trifluoroacetate (7.2 µmol, 2.0 eq.) are dissolved in 0.5 ml acetone and 0.5 ml acetonitrile, 50 mg molecular sieve 3Å is added. After 30 min 5.8 µl 2-Cyanoethyl N,N,N',N'-tetraisopropylphosphordiamidite (18.1 µmol, 5.0 eq.) is added to the stirred solution. The reaction is complete after 5 h. Yield (determined by HPLC): 71%.

### Example 12

### Synthesis of 5'-O-DMTr-dT 3'-O-posphoramidite via in situ generation of N-Methylimidazolium trifluoroacetate

1.00 g 5'-*O*-DMTr-dT-3'-OH (1.84 mmol, 1.0 eq.), is dissolved in 2 mL dichloromethane and 2 mL acetone. 300 mg N-Methylimidazole (3.68 mmol, 291 µL, 2.0 eq.) and 665 mg 2-cyanoethyl-*N,N,N',N'*-tetraisopropylphosphordiamidite (2.21 mmol, 700 µL, 1.2 eq.) followed by 1.00 g molecular sieve 3Å are added. To this stirred suspension 230 mg trifluoracetic acid (2.02 mmol, 159 µL, 1.1 eq.) in 1 mL dichloromethane are added drop wise. The reaction is complete after 3h. Yield (determined by HPLC): 99%

### Example 13

### Synthesis of 5'-O-DMTr-dG^{iBu}-3'-O-posphoramidite via in situ generation of N-Methylimidazolium trifluoroacetate

1.00 g 5'-O-DMTr-dG*^{iBu}*-3'-OH (1.56 mmol, 1.0 eq.), is dissolved in 2 mL dichloromethane and 2 mL acetone. 255 mg N-Methylimidazole (3.11 mmol, 247 µL, 2.0 eq.) and 563 mg 2-cyanoethyl-*N,N,N',N*'-tetraisopropylphosphordiamidite (1.87 mmol, 593 µL, 1.2 eq.) followed by 1.00 g molecular sieve 3Å are added. To this stirred suspension 195 mg trifluoracetic acid (1.72 mmol, 135 µL, 1.1 eq.) in 1 mL dichloromethane are added drop wise. The reaction is complete after 5h. Yield (determined by HPLC): 88 %

### Example 14

### Synthesis of 5'-O-DMTr-dG^{iBu}-3'-O-posphoramidite using N-methylimidazolium trifluoroacetate - N-Methylimidazole mixture

1.00 g 5'-O-DMTr-dG*^{iBu}*-3'-OH (1.56 mmol, 1.0 eq.), is dissolved in 2 mL dichloromethane and 2 mL acetone. 2.00 g molecular sieve 3Å, 367 mg N-methylimidazolium trifluoroacetate (1.87 mmol, 1.2 eq.) and 383 mg N-Methylimidazole (4.68 mmol, 371 µL, 3.0 eq.) are added followed by 563 mg 2-cyanoethyl-*N,N,N',N*'-tetraisopropylphosphordiamidite (1.87 mmol, 593 µL, 1.2 eq.). The reaction is complete after 20 min. Yield (determined by HPLC): 90%

### Example 15

### Synthesis of 5'-O-DMTr-dC^{Bz}-3'-O-posphoramidite using N-methylimidazolium trifluoroacetate - N-Methylimidazole mixture

1.00 g 5'-O-DMTr-dG*^{iBu}*-3'-OH (1.56 mmol, 1.0 eq.), is dissolved in 2 mL dichloromethane and 2 mL acetone. 2.00 g molecular sieve 3Å, 367 mg N-methylimidazolium trifluoroacetate (1.87 mmol, 1.2 eq.) and 383 mg N-Methylimidazole (4.68 mmol, 371 µL, 3.0 eq.) are added followed by 563 mg 2-cyanoethyl-*N,N,N',N'*-tetraisopropylphosphordiamidite (1.87 mmol, 593 µL, 1.2 eq.). The reaction is complete after 20 min. Yield (determined by HPLC): 90%

### Example 16

### Synthesis of 5'-O-DMTr-dC^{Bz}-P(O)-dA^{Bz}-3'-O-posphoramidite via in situ generation of Methylimidazolium trifluoroacetate

100 mg 5'-O-DMTr-dC^{Bz}-P(O)-dA^{Bz}-3'-OH (90.7 µmol, 1.0 eq.), is dissolved in 200 µL dichloromethane and 200 µL acetone. 15 mg N-Methylimidazole (180 µmol, 14 µL, 2.0 eq.) and 54.6 mg 2-cyanoethyl-*N,N,N',N'-*tetraisopropylphosphordiamidite (181 µmol, 57 µL, 2.0 eq.) followed by 100 mg molecular sieve 3Å are added. To this stirred suspension 100 µL of an 1M trifluoracetic acid solution in dichloromethane are added drop wise. The reaction is complete after 30 min. Yield (determined by HPLC): 90 %

### Example 17

### Synthesis of 5'-O-phosphoramidite-dT-P(O)-dG^{iBu}-P(O)-dG^{iBu}-3'-O-Lev using N-methylimidazolium trifluoroacetate

2.0 g 5'-HO-dT-P(O)-dG^{iBu}-P(O)-dG^{iBu}-3'-O-Lev (1.6 mmol, 1.0 eq.) were dissolved in 80 mL acetone, 500 mg methylimidazolium trifluoroacetate (2.5 mmol, 1.56 eq.) and 4.0 g molecular sieve 3Ǻ were added. 2.76 ml 2-cyanoethyl-*N,N,N',N*'-tetraisopropylphosphordiamidite (2.62 g, 8.7 mmol, 5 eq.) were added and after 30 min stirring the phosphoramidite was precipitated by addition of 300 mL n-heptane. Yield (determined by HPLC): 72 %

### Example 18

### Synthesis of 5'-O-phosphoramidite-dC^{Bz}-P(O)-dA^{Bz}-3'-O-Lev using N-methylimidazolium trifluoroacetate

1.0 g 5'-HO-dC^{Bz}-P(O)-dA^{Bz}-3'-*O*-Lev (1.1 mmol, 1.0 eq.) and 326 mg methylimidazolium trifluoroacetate (1.66 mmol, 1.5 eq.) were dissolved in 8 mL acetone and 10 mL dichloromethane and 2.0 g molecular sieve 3 Ǻ were added. 700 µL 2-cyanoethyl-*N,N,N',N'*-tetraisopropylphosphordiamidite (664 mg, 2.2 mmol, 2 eq.) were added and after 1h stirring the phosphoramidite was precipitated by addition of 50 mL n-heptane. Yield (determined by HPLC): 78 %

### Example 19

### Synthesis of 5'-O-phosphoramidite-T-P(O)-dC^{Bz}-P(O)-dC^{Bz}-P(O)-dC^{Bz}-3'-O-Lev using N-methylimidazolium trifluoroacetate

20 mg 5'-HO-T-P(O)-dC^{Bz}-P(O)-dC^{Bz}-P(O)-dC^{Bz}-3'-*O*-Lev (11.6 µmol, 1.0 eq.) and 4.3 mg N-methylimidazolium trifluoroacetate (22 µmol, 1.9 eq.) were dissolved in 2 mL acetone and 40 mg molecular sieve 3 Ǻ were added. 15 µL 2-cyanoethyl-*N,N,N',N'*-tetraisopropylphosphordiamidite (14 mg, 47 µmol, 4 eq.) were added and after 1h stirring the phosphoramidite was precipitated by addition of 3 mL n-heptane. Yield (determined by HPLC): 86 %

### Example 20

### Synthesis of 5'-O-TBDPS-dT-3'-O-posphoramidite using N-Methylimidazolium trifluoroacetate

510 mg 5'-*O*-DMTr-dT-3'-OH (1.06 mmol, 1.0 eq.) are dissolved in 20 mL acetone and 251 N-methylimidazolium trifluoroacetate (1.27 mmol, 1.2 eq), 1.0 g molecular sieve 3Ǻ and 383 mg 2-cyanoethyl-*N,N,N',N*'-tetraisopropylphosphordiamidite (403 µL, 1.27 mmol, 1.2 eq.) are added under stirring. The reaction is complete after 30 min. Yield (determined by HPLC): 88%

### Example 21

### Synthesis of 5'-O-TBDMS-dG^{iBu}-3'-O-posphoramidite using N-Methylimidazolium trifluoroacetate

1 mg 5'-O-TBDMS-dG^{iBu}-3'-OH (2.21 mmol, 1.0 eq.) are dissolved in 20 mL acetone and 875 N-methylimidazolium trifluoroacetate (4.42 mmol, 2 eq), 2.0 g molecular sieve 3Ǻ and 3.33 g 2-cyanoethyl-*N,N,N',N*'-tetraisopropylphosphordiamidite (3.5 mL, 11 mmol, 5 eq.) are added under stirring. The reaction is complete after 30 min. Yield (determined by HPLC): 88%

## Claims

1. A method for preparing a phosphitylated compound comprising the step of:
- reacting a hydroxyl containing compound with a phosphitylating agent in the presence of an activator having the formula I 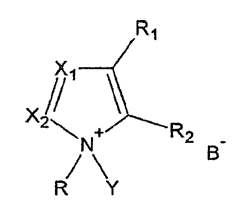 wherein
R = alkyl, cycloalkyl, aryl, aralkyl, heteroalkyl, heteroaryl
R₁, R₂ = either H or form a 5 to 6-membered ring together.
X₁, X₂ = independently either N or CH
Y = H or Si(R₄)₃, with R₄= alkyl, cycloalkyl, aryl, aralkyl, heteroalkyl, heteroaryl
B = deprotonated acid.

2. The method of claim 1, wherein: the phosphitylated compound is a phosphoramidite.

3. The method of claim 1 or 2, wherein the activator has a formula selected from the group consisting of 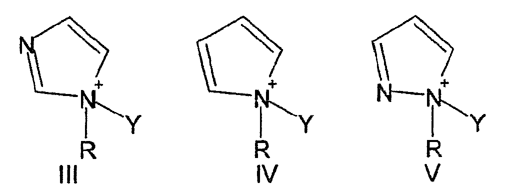 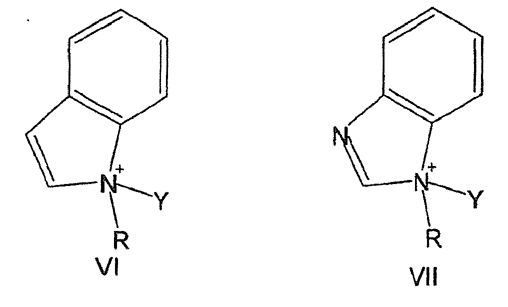 wherein
Y is defined as in claim 1
R is methyl, phenyl or benzyl.

4. The method of any one of claims 1 to 3, wherein the hydroxyl containing compound comprises a sugar moiety.

5. The method of any one of claims 1 to 4, wherein the hydroxyl containing compound is a nucleoside or an oligomer derived there from.

6. The method of any one of claims 1 to 5, wherein the hydroxyl containing compound is a 5'-O-protected nucleoside having a 3'-hydroxyl group or a 3'-O-protected nucleoside having a 5'-hydroxyl group.

7. The method of any one of claims 1 to 6, wherein the activator is prepared in-situ and used without purification.

8. The method of any one of claims 1 to 7, wherein the reaction is in the presence of a mixture of the activator having the formula I and a corresponding base with formula VIII 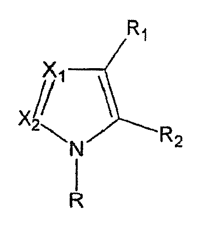 wherein R₁, R₂, X₁, X₂ and R are defined as in claim 1.

9. The method of claim 8, wherein the corresponding base is brought into contact with the hydroxyl containing compound and the phosphitylating agent and an acid H⁺B⁻ is added.

10. The method of any one of claims 1 to 9, wherein the phosphitylating agent has the formula II 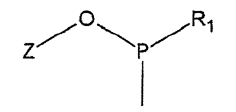 wherein Z represents a leaving group and R₁ and R₂ are independently secondary amino groups or halogen atoms.

11. The method of any one of claims 1 to 10, wherein the phosphitylating agent is 2-cyanoethyl-N,N,N',N'-tetraisopropylphosphorodiamidite.

12. The method of any one of claims 1 to 11, wherein the deprotonated acid is derived from the group consisting of trifluoroacetic acid, dichloroacetic acid, methane sulfonic acid, trifluormethane sulfonic acid (triflate), o-chlorophenolate and mixtures thereof.

13. The method of any one of claims 1 to 12, wherein the reaction is in the presence of acetone.

14. The use of an activator having formula I 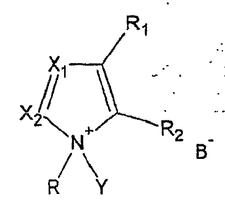 wherein
R = alkyl, cycloalkyl, aryl, aralkyl, heteroalkyl, heteroaryl
R₁, R₂ = either H or form a 5 to 6-membered ring together.
X₁, X₂ = independently either N or CH
Y = H or Si(R₄)₃, with R₄= alkyl, cycloalkyl, aryl, aralkyl, heteroalkyl, heteroaryl
B = deprotonated acid
as an activator for phoshitylating hydroxyl containing compounds with a phosphitylating agent.

15. The use of claim 14 for the synthesis of adenosine phosphoramidite, cytosine phosphoramidite, guanosine phosphoramidite and uracil phosphoramidite, desoxyadenosine phosphoramidite, desoxyguanosine phosphoramidite, desoxythymidin phosphoramidte, desoxycytosine phosphoramidite as well as oligonucleotide phosphoramidates having the formula Xₙ, wherein each X is selected from A, dA, C, dC, G, dG, U, dT and n = 2 to 8, preferably 2 to 6 and derivatives thereof comprising protective groups.

16. A mixture of an activator having formula I 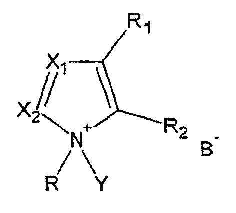 wherein
R = alkyl, cycloalkyl, aryl, aralkyl, heteroalkyl, heteroaryl
R₁, R₂ = either H or form a 5 to 6-membered ring together.
X₁, X₂ = independently either N or CH
Y = H or Si(R₄)₃, with R₄= alkyl, cycloalkyl, aryl, aralkyl, heteroalkyl, heteroaryl
B = deprotonated acid
and with an additive having formula VIII 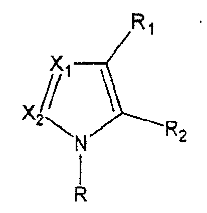 wherein R, R₁, R₂, X₁, X₂, are defined as for formula I
or pyridine in a molar ratio of 1:1 to 1:10 (mol:mol).

17. Use of the mixture of claim 16 for the synthesis of adenosine phosphoramidite, cytosine phosphoramidite, guanosine phosphoramidite and uracil phosphoramidite, desoxyadenosine phosphoramidite, desoxyguanosine phosphoramidite, desoxythymidin phosphoramidte, desoxycytosine phosphoramidite as well as oligonucleotide phosphoramidates having the formula Xₙ, wherein each X is selected from A, dA, C, dC, G, dG, U, dT and n = 2 to 8, preferably 2 to 6 and derivatives thereof comprising protective groups.

## Patentansprüche

1. Verfahren zur Herstellung einer phosphitylierten Verbindung, bei dem man : eine hydroxylgruppenhaltige Verbindung in Gegenwart eines Aktivators der Formel I 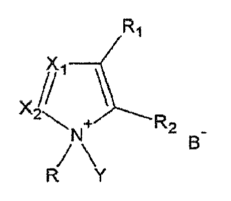 worin
R = Alkyl, Cycloalkyl, Aryl, Aralkyl, Heteroalkyl, Heteroaryl
R₁, R₂ = entweder H oder zusammen einen 5- bis 6-gliedrigen Ring bilden
X₁, X₂ = unabhängig voneinander N oder CH
Y = H oder Si(R₄)₃, mit R₄ = Alkyl, Cycloalkyl, Aryl, Aralkyl, Heteroalkyl, Heteroaryl
B = deprotonierte Säure,
mit einem Phosphitylierungsmittel umsetzt.

2. Verfahren nach Anspruch 1, bei dem es sich bei der phosphitylierten Verbindung um ein Phosphoramidit handelt.

3. Verfahren nach Anspruch 1 oder 2, bei dem der Aktivator eine Formel aus der Gruppe bestehend aus 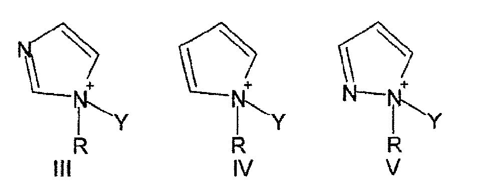 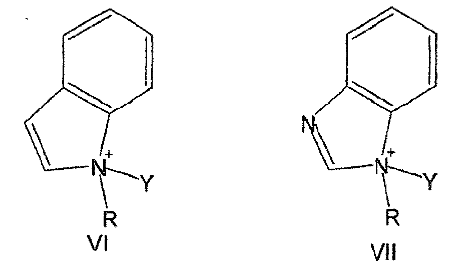 worin
Y wie in Anspruch 1 definiert ist
R für Methyl, Phenyl oder Benzyl steht,
aufweist.

4. Verfahren nach einem der Ansprüche 1 bis 3, bei dem die hydroxylgruppenhaltige Verbindung eine Zucker gruppierung umfasst.

5. Verfahren nach einem der Ansprüche 1 bis 4, bei dem es sich bei der hydroxylgruppenhaltigen Verbindung um ein Nukleosid oder ein davon abgeleitetes Oligomer handelt.

6. Verfahren nach einem der Ansprüche 1 bis 5, bei dem es sich bei der hydroxylgruppenhaltigen Verbindung um ein 5'-O-geschütztes Nukleosid mit einer 3'-Hydroxylgruppe oder ein 3'-O-geschütztes Nukleosid mit einer 5'-Hydroxylgruppe handelt.

7. Verfahren nach einem der Ansprüche 1 bis 6, bei dem man den Aktivator in situ herstellt und ohne Reinigung verwendet.

8. Verfahren nach einem der Ansprüche 1 bis 7, bei dem die Umsetzung in Gegenwart einer Mischung des Aktivators mit der Formel I und einer korrespondierenden Base der Formel VIII 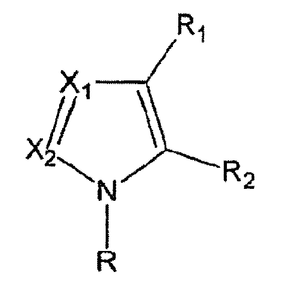 worin R₁, R₂, X₁, X₂ und R wie in Anspruch 1 definiert sind, erfolgt.

9. Verfahren nach Anspruch 8, bei dem man die korrespondierende Base mit der hydroxylgruppen haltigen Verbindung und dem Phosphitylierungs mittel in Berührung bringt und eine Säure H⁺B⁻ zugibt.

10. Verfahren nach einem der Ansprüche 1 bis 9, bei dem das Phosphitylierungsmittel die Formel II 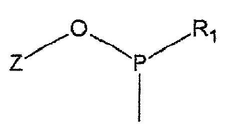 worin Z für eine Abgangsgruppe steht und R₁ und R₂ unabhängig voneinander für sekundäre Aminogruppen oder Halogenatome stehen, aufweist.

11. Verfahren nach einem der Ansprüche 1 bis 10, bei dem es sich bei dem Phosphitylierungs mittel um 2-Cyanoethyl-N,N,N',N'-tetraisopropylphosphoramidit handelt.

12. Verfahren nach einem der Ansprüche 1 bis 11, bei dem sich die deprotonierte Säure von der Gruppe bestehend aus Trifluoressigsäure, Dichloressig säure, Methansulfonsäure, Trifluormethan sulfon säure (Triflat), o-Chlorphenolat und Mischungen davon ableitet.

13. Verfahren nach einem der Ansprüche 1 bis 12, bei dem die Umsetzung in Gegenwart von Aceton erfolgt.

14. Verwendung eines Aktivators der Formel I 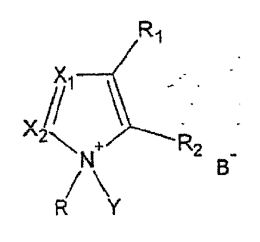 worin
R = Alkyl, Cycloalkyl, Aryl, Aralkyl, Heteroalkyl, Heteroaryl
R₁, R₂ = entweder H oder zusammen einen 5- bis 6-gliedrigen Ring bilden
X₁, X₂ = unabhängig voneinander N oder CH
Y = H oder Si(R₄)₃, mit R₄ = Alkyl, Cycloalkyl, Aryl, Aralkyl, Heteroalkyl, Heteroaryl
B = deprotonierte Säure,
als Aktivator zur Phosphitylierung von hydroxyl gruppenhaltigen Verbindungen mit einem Phosphitylie rungsmittel.

15. Verwendung nach Anspruch 14 zur Synthese von Adenosinphosphoramidit, Cytosinphosphoramidit, Guanosinphosphoramidit und Uracilphosphoramidit, Des oxyadenosinphosphoramidit, Desoxyguanosinphosphor amidit, Des oxythymidinphosphoramidit, Desoxycytosin phosphoramidit sowie Oligonucleotid phosphoramiditen der Formel Xₙ, worin X jeweils aus A, dA, C, dC, G, dG, U, dT ausgewählt ist und n gleich 2 bis 8, vorzugsweise 2 bis 6, und Schutzgruppen enthaltenden Derivaten davon.

16. Mischung eines Aktivators der Formel I 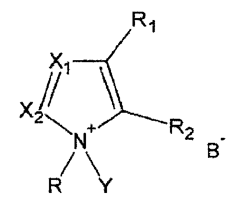 worin
R = Alkyl, Cycloalkyl, Aryl, Aralkyl, Heteroalkyl, Heteroaryl
R₁, R₂ = entweder H oder zusammen einen 5- bis 6-gliedrigen Ring bilden
X₁, X₂ = unabhängig voneinander N oder CH
Y = H oder Si(R₄)₃, mit R₄ = Alkyl, Cycloalkyl, Aryl, Aralkyl, Heteroalkyl, Heteroaryl
B = deprotonierte Säure,
mit einem Additiv der Formel VIII 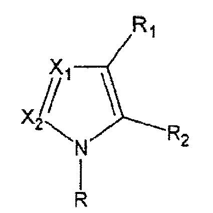 worin R₁, R₂, X₁, X₂ und R wie für Formel I definiert sind,
oder Pyridin in einem Molverhältnis von 1:1 1 bis 1:10 (mol:mol).

17. Verwendung der Mischung nach Anspruch 16 zur Synthese von Adenosinphosphoramidit, Cytosin phosphoramidit, Guanosinphosphoramidit und Uracil phosphoramidit, Des oxyadenosinphosphoramidit, Desoxyguanosinphosphor amidit, Des oxythymidin phosphor amidit, Desoxycytosin phosphoramidit sowie Oligonucleotid phosphoramiditen der Formel Xₙ, worin X jeweils aus A, dA, C, dC, G, dG, U, dT ausgewählt ist und n gleich 2 bis 8, vorzugsweise 2 bis 6, und Schutzgruppen enthaltenden Derivaten davon.

## Revendications

1. Procédé pour préparer un composé phosphitylé comprenant l'étape de : réaction d'un composé contenant hydroxyle avec un agent de phosphitylation en présence d'un activateur ayant la formule I 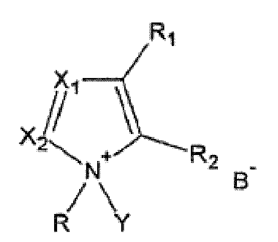 dans laquelle
R = alkyle, cycloalkyle, aryle, aralkyle, hétéroalkyle, hétéroaryle
R₁, R₂ = soit H ou forment un cycle de 5 à 6 chaînons conjointement.
X₁, X₂ =indépendamment soit N ou CH
Y = H ou Si(R₄)₃, avec R₄ = alkyle, cycloalkyle, aryle, aralkyle, hétéroalkyle, hétéroaryle
B =acide déprotoné.

2. Procédé de la revendication 1, dans lequel : le composé phosphitylé est un phosphoramidite.

3. Procédé de la revendication 1 ou 2, dans lequel l'activateur a une formule choisie dans le groupe constitué de 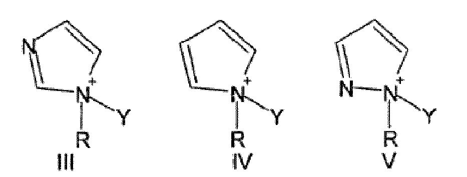 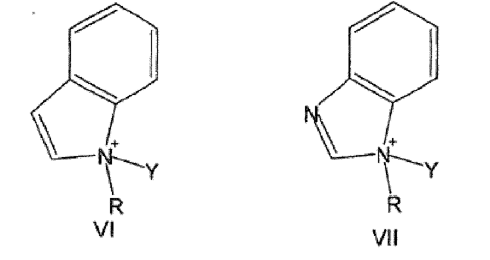 dans laquelle
Y est tel que défini dans la revendication 1
R est méthyle, phényle ou benzyle.

4. Procédé de l'une quelconque des revendications 1 à 3, dans lequel le composé contenant hydroxyle comprend un fragment glucidique.

5. Procédé de l'une quelconque des revendications 1 à 4, dans lequel le composé contenant hydroxyle est un nucléoside ou un dérivé oligomère de celui-ci.

6. Procédé de l'une quelconque des revendications 1 à 5, dans lequel le composé contenant hydroxyle est un nucléoside 5'-O-protégé ayant un groupe 3'-hydroxyle ou un nucléoside 3'-O-protégé ayant un groupe 5'-hydroxyl.

7. Procédé de l'une quelconque des revendications 1 à 6, dans lequel l'activateur est préparé *in situ* et utilisé sans purification.

8. Procédé de l'une quelconque des revendications 1 à 7, dans lequel la réaction est conduite en présence d'un mélange de l'activateur ayant la formule I et une base correspondante ayant la formule VIII 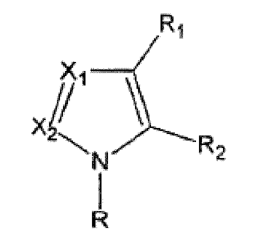 dans laquelle R₁, R₂, X₁, X₂ et R sont tels que définis dans la revendication 1.

9. Procédé de la revendication 8, dans lequel la base correspondante est mise en contact avec le composé contenant hydroxyle et l'agent de phosphitylation et un acide H⁺B⁻ est ajouté.

10. Procédé de l'une quelconque des revendications 1 à 9, dans lequel l'agent de phosphitylation a la formule II 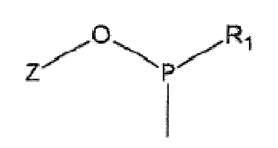 dans laquelle Z représente un groupe partant et R₁ et R₂ sont indépendamment des groupes amino secondaires ou des atomes d'halogène.

11. Procédé de l'une quelconque des revendications 1 à 10, dans lequel l'agent de phosphitylation est le 2-cyanoéthyl-N,N,N',N'-tétra-isopropylphosphorodiamidite.

12. Procédé de l'une quelconque des revendications 1 à 11, dans lequel l'acide déprotoné est dérivé du groupe constitué de l'acide trifluoroacétique, l'acide dichloroacétique, l'acide méthanesulfonique, l'acide trifluorométhanesulfonique (triflate), le o-chlorophénolate et des mélanges de ceux-ci.

13. Procédé de l'une quelconque des revendications 1 à 12, dans lequel la réaction est conduite en présence d'acétone.

14. Utilisation d'un activateur ayant la formule I 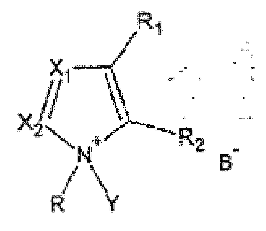 dans laquelle
R = alkyle, cycloalkyle, aryle, aralkyle, hétéroalkyle, hétéroaryle
R₁, R₂ = soit H ou forment un cycle de 5 à 6 chaînons conjointement
X₁, X₂ = indépendamment soit N ou CH
Y = H ou Si(R₄)₃, avec R₄ = alkyle, cycloalkyle, aryle, aralkyle, hétéroalkyle, hétéroaryle
B = acide déprotoné
en tant qu'activateur pour phosphityler des composés contenant hydroxyle avec un agent de phosphitylation.

15. Utilisation de la revendication 14 pour la synthèse d'adénosine phosphoramidite, de cytosine phosphoramidite, de guanosine phosphoramidite et d'uracile phosphoramidite, de désoxyadénosine phosphoramidite, de désoxyguanosine phosphoramidite, de désoxythymidine phosphoramidite, de désoxycytosine phosphoramidite ainsi que des phosphoramidates d'oligonucléotide ayant la formule Xₙ, chaque X étant choisi parmi A, dA, C, dC, G, dG, U, dT et n = 2 à 8, de préférence de 2 à 6 et des dérivés de ceux-ci comprenant des groupes protecteurs.

16. Mélange d'un activateur ayant la formule I 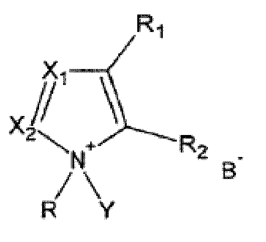 dans laquelle
R= alkyle, cycloalkyle, aryle, aralkyle, hétéroalkyle, hétéroaryle
R₁, R₂ =soit H ou forment un cycle de 5 à 6 chaînons conjointement.
X₁, X₂ = indépendamment N ou CH
Y = H ou Si(R₄)₃, avec R₄ = alkyle, cycloalkyle, aryle, aralkyle, hétéroalkyle, hétéroaryle
B = acide déprotoné
et avec un additif ayant la formule VIII 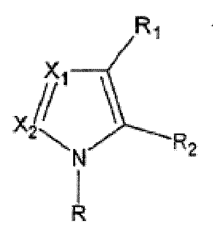 dans laquelle R, R₁, R₂, X₁, X₂, sont définis comme pour la formule I
ou la pyridine dans un rapport molaire de 1:1 à 1:10 (mole:mole).

17. Utilisation du mélange de la revendication 16 pour la synthèse d'adénosine phosphoramidite, de cytosine phosphoramidite, de guanosine phosphoramidite et d'uracile phosphoramidite, de désoxyadénosine phosphoramidite, de désoxyguanosine phosphoramidite, de désoxythymidine phosphoramidite, de désoxycytosine phosphoramidite ainsi que des phosphoramidates d'oligonucléotide ayant la formule Xₙ, dans laquelle chaque X est choisi parmi A, dA, C, dC, G, dG, U, dT et n = 2 à 8, de préférence 2 à 6 et des dérivés de ceux-ci comprenant des groupes protecteurs.
